# EUROPEAN PATENT APPLICATION

(11) **EP 1 634 533 A1**
(43) Date of publication of application: **15.03.2006**
(21) Application number: 04746033.2
(22) Date of filing: 10.06.2004
(51) Int. Cl.: A61B 5/107

(54) **OPTICAL FAT MEASURING DEVICE**

(30) Priority: 13.06.2003 JP 2003169619
(71) Applicant: MATSUSHITA ELECTRIC INDUSTRIAL CO., LTD., Kadoma-shi, Osaka 571-8501 (JP)
(72) Inventor: KONDOH, Kazuya, Osaka 576-0022 (JP); UCHIDA, Shinji, Osaka 572-0807 (JP)
(74) Representative: Grünecker, Kinkeldey, Stockmair & Schwanhäusser Anwaltssozietät
(86) International application number: PCT/JP2004/008518
(87) International publication number: WO 2004/110273

(57) **Abstract**

Measurement accuracy decreases if the sensitivity of a light source or a light receiving element is degraded owing to contamination or the like.

After a waveguide 14 has been placed opposite a light source 2 and a light receiving section 3, a reference value for the quantity of light received predetermined for each light receiving section 3 is compared with the quantity of received light which has been guided from the lighted light source section 2 to the plurality of light receiving sections 3 through the waveguide 14. If the quantities of light received by a number of light receiving sections 3 are smaller than the reference value for the quantity of light received corresponding to these light receiving sections, the number of these light receiving sections 3 being smaller than that of the plurality of light receiving section 3, the need to clean the light receiving section is displayed. If the quantities of light received by all of the plurality of light receiving sections 3 are smaller than the reference value for the quantity of light received corresponding to these light receiving sections 3, the need to clean the light source 2 is displayed.

## Description

### Technical Field

The present invention relates to an optical fat measuring apparatus capable of optically measuring subcutaneous fat, as well as a standard element.

### Background Art

In the prior art, there is a method of measuring the thickness of subcutaneous fat in a living body by receiving a part of light emitted by a light source placed on the surface of the living body and entering the living body, the part propagating through the living body while being scattered and absorbed and then appearing on the surface of the living body again (refer to, for example, Japanese Patent Laid-Open No. 2000-155091).

With this method, a light source section and a light receiving section are arranged on the skin in a straight line, and the light source emits a near infrared light. Then, the light receiving section receives a light transmitted through the fat layer. The quantity of light received by the light receiving section is applied to a calibration curve for the quantity of light received and the thickness of the fat to estimate the thickness of the fat.

A light propagation characteristic varies significantly between the muscle and the fat. More light is absorbed by the muscle, whereas light is more markedly scattered by the fat. This difference in characteristic is significant for a light having a wavelength of 500 to 1,000 nm. Thus, the thicker the subcutaneous fat layer is, the more widely the light emitted by the light source and entering the surface of the living body is spread. The light is diffusively spread not only in the direction of the depth but also in a lateral direction. Accordingly, the quantity of light laterally spread and exiting the surface of the living body again increases consistently with the thickness of the subcutaneous fat. Therefore, the thickness and amount of subcutaneous fat can be measured by using the light receiving section to receive light.

Further, with this method, the light source section and the light receiving section are arranged so as to obtain a plurality of transmitted to received height distances, in order to correct the color of the skin or the like. This makes it possible to accurately measure the thickness of the subcutaneous fat. Specifically, when the quantity of light received by the light receiving section is determined, the quantity of light received by each light receiving section, which is dependent on the skin color difference, is corrected by the quantity of light received by the light receiving element closest to the light source section.

There is also a method of providing a reflector on a protective cover to obtain reference data on the percent of body fat as a reference (refer to, for example, Japanese Patent No. 2648377). With this method, a protective cover is placed so that a surface of the protective cover which has a reflector lies opposite a surface of the device which has a light source section and a light receiving section. The light source section emits light, and the reflector of the protective cover reflects the light. Then, the quantity of light incident on the light receiving section is measured. The measured quantity of light received is used as reference data on the percentage of body fat.

However, with the above conventional fat measuring apparatus, according to the method described in Japanese Patent Laid-Open No. 2000-155091, measurement accuracy decreases if the light source or the light receiving element has a reduced sensitivity owing to contamination.

That is, because the light source used is a near infrared light, which is less visible tohumanbeings, even if an emission end of the light source section is contaminated and thus has a significantly reduced output, the user fails to notice this while using the device. As a result, disadvantageously, the accuracy decreases.

Further, if there are a plurality of light receiving sections and if the incidence ends of only some light receiving sections are contaminated, corrections for the skin may not be made. This also reduces the measurement accuracy.

Furthermore, the method according to Japanese Patent No. 2648377 uses the reflector attached to the protective cover. This calibration is effective if the light receiving section and the light source are located close to each other or at the same transmitted to received light distance. However, with a measurement system with a plurality of transmitted to received light distances, which are different from one another, a signal level resulting from measurement of the living body varies exponentially with the transmitted to received light distances. Accordingly, with the simple specified reflector configuration, it is difficult to reproduce the exponentially varying quantity of light.

Description will be given of the case of a plurality of transmitted to received light distances where one light source section and a plurality of light receiving sections are lined up straight. In this case, a light receiving section farther from the light source section has a smaller quantity of light received. In other words, the quantity of light received by the light receiving section is expressed by the exponent of the distance from the light source section to the light receiving section. Therefore, a light receiving section located farther from the light source is pre-adjusted so as to be able to detect a smaller quantity of light.

However, if the protective cover with the reflector described in Japanese Patent No. 2648377 is placed opposite the one light source and plurality of light receiving sections and light emitted by the light source section and then reflected by the reflector is received by the light receiving sections, the quantity of light received by the light receiving section is almost the same for all the light receiving sections. Accordingly, a light receiving section placed farther from the light source section has an excessively large amount of incident light and may thus be saturated. In contrast, if the quantity of light is adjusted for the light receiving section located farthest from the light source section to receive an optimal quantity of incident light, a light receiving section placed closer to the light source section has an excessively small quantity of incident light and cannot sense the quantity of incident light. With such a simple reflector configuration, it is difficult to reproduce an exponentially varying quantity of light.

### Disclosure of the Invention

In view of the problems, it is an object of the present invention to provide an optical fat measuring apparatus and a standard element that can correct a measurement error caused by a change in temperature or a secular change by checking the sensitivities of a light, source and a light receiving element.

In view of the problems, it is an object of the present invention to provide an optical fat measuring apparatus and a standard element that enables the amount of fat to be always accurately and reproducibly measured by sensing the contamination of the light source and light receiving element to display the need for cleaning.

A first invention is an optical fat measuring apparatus comprising:
a light source section and a light receiving section arranged so as to obtain a plurality of transmitted to received light distances;
an arithmetic section which calculates information on fat in a living body on the basis of the quantity of light received by said light receiving section, the light being emitted by said light source section and propagating through the living body; and
a standard element having a waveguide which guides light from said light source section to said light receiving section when placed opposite said light source section and said light receiving section, said waveguide having a predetermined transmittance, and
wherein at least one of said light source section and said light receiving section, is arranged plurally.

A second invention is the optical fat measuring apparatus according to the first invention, having one of said light source sections and a plurality of said light receiving sections, and
wherein said apparatus comprises an operation checking section which compares a reference value for the quantity of light received predetermined for each said light receiving section with the quantity of received light which has been guided from lighted said light source section to the plurality of said light receiving sections through said waveguide after said standard element has been placed so that said waveguide is opposite said light source section and said light receiving sections,
which operates if the quantities of light received by a smaller number of said light receiving sections than that of the plurality of said light receiving sections are smaller than said reference value for the quantity of light received corresponding to these light receiving sections, to determine that these light receiving sections are defective, and
which operates if each quantities of light received by all of the plurality of said light receiving sections are smaller than the reference value for the quantity of light received corresponding to said each light receiving sections, to determine that said light source section is defective.

A third invention is the optical fat measuring apparatus according to the first invention, wherein if said operation checking section determines that said light receiving section is defective, said operation checking section shows that said light receiving section determined to be defective must be cleaned or provides a corresponding sound output, and
if said operation checking section determines that said light source section is defective, said operation checking section shows that said light source section determined to be defective must be cleaned or provides a corresponding sound output.

A fourth invention is the optical fat measuring apparatus according to the first invention, having a plurality of said light source sections and one said light receiving section, and
wherein the apparatus comprises an operation checking section which compares a reference value for the quantity of light receivedpredetermined for each said light source section with the quantity of received light which has been guided from the independently lighted plurality of said light source sections to said light receiving section through said waveguide after said standard element has been placed so that said waveguide is opposite said light source sections and said light receiving section,
which operates if the quantities of light received from a smaller number of said light source sections than that of the plurality of said light source sections are smaller than said reference value for the quantity of light received corresponding to these light source sections, to determine that these light source sections are defective, and
which operates if each quantities of light received from all of the plurality of said light source sections are smaller than the reference value for the quantity of light received corresponding to said each light source sections, to determine that said light receiving section is defective.

A fifth invention is the optical fat measuring apparatus according to the first invention, wherein if said operation checking section determines that said light source section is defective, said operation checking section shows that said light source section determined to be defective must be cleaned or provides a corresponding sound output, and
if said operation checking section determines that said light receiving section is defective, said operation checking section shows that said light receiving section determined to be defective must be cleaned or provides a corresponding sound output.

A sixth invention is the optical fat measuring apparatus according to the first invention, having one of said light source section and a plurality of said light receiving sections, and
wherein said arithmetic section has a correcting section which corrects measured values for said living body using the quantity of received light which has been guided from lighted said light source section to the plurality of said light receiving sections through said waveguide after said standard element has been placed so that said waveguide is opposite said light source section and said light receiving sections.

A seventh invention is the optical fat measuring apparatus according to the first invention, having a plurality of said light source sections and one said light receiving section, and
wherein said arithmetic section has a correcting section which corrects measured values for said living body using the quantity of received light which has been guided from independently lighted plurality of said light source sections to said light receiving section through said waveguide after said standard element has been placed so that said waveguide is opposite said light source sections and said light receiving section.

A eighth invention is the optical fat measuring apparatus according to the sixth invention, wherein said light receiving section includes a first light receiving section and a second light receiving section, and
after said standard element has been placed so that said waveguide is opposite said light source section and said light receiving sections, said correcting section corrects the measured values for said living body on the basis of the ratio of the quantity of light received by said first light receiving section, the light being guided from lighted said light source and to said first light receiving section through said waveguide, to the quantity of light received by said second light receiving section, the light being guided from lighted said light source to said second light receiving section through said waveguide.

A ninth invention is the optical fat measuring apparatus according to the seventh invention, wherein said light source section includes a first light source section and a second light source section, and
after said standard element has been placed so that said waveguide is opposite said light source sections and said light receiving section, said correcting section corrects the measured values for said living body on the basis of the ratio of the quantity of received light guided from independently lighted said first light source to said light receiving section through said waveguide, to the quantity of received light guided from independently lighted said second light source to said light receiving section through said waveguide.

A tenth invention is the optical fat measuring apparatus according to anyone of the first to the ninth invention, wherein said standard element is connected by a rotating shaft to a main body with said light source section and said light receiving section.

An eleventh invention is a standard element comprising a waveguide which can be placed opposite a light source section and a light receiving section of the optical fat measuring apparatus and which guides light from said light source section to said light receiving section when placed opposite said light source section and said light receiving section of said optical fat measuring apparatus, said waveguide having a predetermined transmittance.

A twelfth invention is the standard element according to the eleventh invention wherein said waveguide is a scatterer which scatters light or an absorber which absorbs light.

A thirteenth invention is the standard element according to the eleventh invention wherein a reflecting layer is provided in the parts of said waveguide other than its surface opposite said light source section and said light receiving section when said standard element is placed so that said waveguide is opposite said light source section and said light receiving section.

A fourteenth invention is the standard element according to the eleventh invention wherein a space is provided between said waveguide and said light source section and said light receiving section when said standard element is placed so that said waveguide is opposite said light source section and said light receiving section.

A fifteenth invention is the standard element according to the fourteenth invention wherein said waveguide partly has a concave portion and said concave portion forms said space.

A sixteenth invention is the standard element according to the eleventh invention wherein said standard element has a protective cover which covers the parts of said waveguide other than its surface opposite said light source section and said light receiving section.

A seventeenth invention is a method of optically measuring fat, said method of making measurement using an optical fat measuring apparatus comprising:
one light source section and a plurality of light receiving sections arranged so as to obtain a plurality of transmitted to received light distances;
an arithmetic section which calculates information on fat in a living body on the basis of the quantity of light received by the plurality of said light receiving sections, the light being emitted by said light source section and propagating through the living body; and
a standard element having a waveguide which guides light from said light source section to said light receiving section when placed opposite said light source section and said light receiving section, said waveguide having a predetermined transmittance, the method comprising:
   a step of comparing a reference value for the quantity of light predetermined for each said light receiving section with the quantity of received light which has been guided from lighted said light source section to the plurality of said light receiving sections through said waveguide after said standard element has been placed around said light source section and said light receiving sections with said waveguide placed opposite said light source section and said light receiving sections; and
   a step of, if the quantities of light received by a smaller number of said light receiving,sections than that of the plurality of said light receiving sections are smaller than said reference value for the quantity of light received corresponding to these light receiving sections, to determine that these light receiving sections are defective, and
   if each quantities of light received by all of the plurality of said light receiving sections are smaller than the reference value for the quantity of light received corresponding to said each light receiving sections, to determine that said light source section is defective.

An eighteenth invention is a method of optically measuring fat, said method of making measurement using an optical fat measuring apparatus comprising:
a plurality of light source sections and one light receiving section arranged so as to obtain a plurality of transmitted to received light distances;
an arithmetic section which calculates information on fat in a living body on the basis of the quantity of light received by the plurality of said light receiving sections, the light being emitted by the independently lighted plurality of said light source sections and propagating through the living body; and
a standard element having a waveguide which guides light from said light source sections to said light receiving section when placed opposite said light source sections and said light receiving section, said waveguide having a predetermined transmittance, said method comprising:
   a step of comparing a reference value for the quantity of light predetermined for each said light source section with the quantity of received light which has been guided from the independently lighted plurality of said light source sections to said light receiving section through said waveguide after said standard element has been placed around said light source sections and said light receiving section with said waveguide placed opposite said light source sections and said light receiving section; and
   a step of, if the quantities of light received from a smaller number of said light source sections than that of the plurality of said light sections are smaller than said reference value for the quantity of light received corresponding to these light source sections, to determine that these light source sections are defective, and
   if the quantities of light received from all of the plurality of said light source sections are smaller than the reference value for the quantity of light received corresponding to these light source sections, determining that said light receiving section is defective.

A nineteenth invention is a method of optically measuring fat, said method of making measurement using an optical fat measuring apparatus comprising:
one light source section and a plurality of light receiving sections arranged so as to obtain a plurality of transmitted to received light distances;
an arithmetic section which calculates information on fat in a living body on the basis of the quantity of light received by the plurality of said light receiving sections, the light being emitted by said light source section and propagating through the living body; and
a standard element having a waveguide which guides light from said light source section to said light receiving section when placed opposite said light source section and said light receiving section, said waveguide having a predetermined transmittance, said method comprising:
   a step of correcting measured values for said living body using the quantity of received light which has been guided from said lighted light source section to the plurality of said light receiving sections through said waveguide after said standard element has been placed around said light source section and said light receiving sections with said waveguide placed opposite said light source section and said light receiving sections.

A twentieth invention is a method of optically measuring fat, said method of making measurement using an optical fat measuring apparatus comprising:
a plurality of light source sections and one light receiving section arranged so as to obtain a plurality of transmitted to received light distances;
an arithmetic section which calculates information on fat in a living body on the basis of the quantity of light received by the plurality of said light receiving sections, said light being emitted by the independently lighted plurality of said light source sections and propagating through said living body; and
a standard element having a waveguide which guides light from said light source sections to said light receiving section when placed opposite said light source sections and said light receiving section, said waveguide having a predetermined transmittance, said method comprising:
   a step of correcting measured values for said living body using the quantity of received light which has been guided from independently lighted plurality of said light source sections to said light receiving section through saidwaveguide after said standard element has been placed around said light source sections and said light receiving section with said waveguide placed opposite said light source sections and said light receiving section.

### Brief Description of the Drawings

Figure 1(a) is a diagram showing the configuration of an optical fat measuring apparatus according to Embodiment 1 of the present invention;
Figure 1(b) is a diagram of the optical fat measuring apparatus according to Embodiment 1 of the present invention as viewed from a living body;
Figure 2 is a graph showing the relationship between the received light quantity ratio of Y1/Y2 and the thickness of subcutaneous fat;
Figure 3 is a diagram showing how a protective cover according to Embodiment 1 of the present invention is attached to a main body; and
Figure 4 is a diagram showing the configuration of an optical fat measuring apparatus according to Embodiment 2 of the present invention.

### (Description of symbols)

- 1: Living body surface
- 2: Light source
- 3: Light receiving section
- 4: Measuring light receiving section
- 5: Correcting light receiving section
- 6: Subcutaneous fat
- 7: Skin
- 8: Arithmetic section
- 9: Display section
- 10: Communication section
- 11: Input section
- 12: Muscle
- 13: Protective cover
- 14: Waveguide
- 15: Recess
- 16: Operation checking section
- 17: Correction section
- 18: Main body
- 19: Rotating shaft
- 20: Cover
- 21: Measuring light source
- 22: Correcting light source
- 23: Reflection layer

### Best Mode for Carrying Out the Invention

An optical fat measuring apparatus according to an embodiment of the present invention comprises one light source section and a plurality of light receiving sections arranged so as to obtain a plurality of transmitted to received light distances. The apparatus calculates information on fat on the basis of the quantity of light received by the light receiving sections with respect to each transmitted to received light distance. The apparatus has a waveguide provided in a protective cover that blocks light, the waveguide having a specified transmittance. Before measurement is started and when an operation checking section is covered with the protective cover, the light source is lighted. The apparatus compares a reference value predetermined as a reference with the quantity of light received by each of the plurality of light receiving sections after the light propagating through the waveguide in the protective cover. Then, if the quantity of light received by one of the light receiving sections is small, the apparatus senses a defect in this light receiving section. The apparatus then shows that this light receiving section must be cleaned or auditorily outputs the need for cleaning. If the quantities of light received by all the light receiving sections are small, the apparatus senses a defect in the light source. The apparatus then shows that this light source must be cleaned or auditorily outputs the need for cleaning. It is thus possible to inform a user of the defect and measures to be taken. Consequently, accurate fat measurements are always possible.

Further, before measurement is started and when a correction section is covered with the protective cover, the light source is lighted. Then, on the basis of the ratio of two quantities of light received by two light receiving sections after the light propagating through the waveguide in the protective cover, degraded accuracy in measured values can be corrected which may result from a variation in the sensitivities of the plurality of light receiving sections caused by a change in temperature and a secular change. As a result, accurate fat measurements are always possible.

Further, an optical fat measuring apparatus according to another embodiment of the present invention comprises a plurality of light source sections and one light receiving section arranged so as to obtain a plurality of transmitted to received light distances. The apparatus calculates information on fat on the basis of the quantity of light received by the light receiving section with respect to each transmitted to received light distance. The apparatus has a waveguide provided in a protective cover that blocks light, the waveguide having a specified transmittance. An operation checking section individually lights each light source. The apparatus compares a reference value predetermined as a reference with the quantity of light received by the light receiving section after the light propagating through the waveguide. Then, if the quantity of light from one light source is small, the apparatus senses a defect in this light source. The apparatus then shows that this light source must be cleaned or auditorily outputs the need for cleaning. If the quantities of light from all the light sources are small, the apparatus senses a defect in the light receiving section. The apparatus then shows that this light receiving section must be cleaned or auditorily outputs the need for cleaning. It is thus possible to inform a user of the defect and measures to be taken. Consequently, accurate fat measurements are always possible.

Further, before measurement is started and when a correction section is covered with the protective cover, each light source is individually lighted. Then, on the basis of the ratio of two quantities of light received by the light receiving section after the light propagating through the waveguide in the protective cover, inaccuracy in measured values can be corrected which may result from a variation among outputs from the plurality of light sources caused by a change in temperature and a secular change. As a result, accurate fat measurements are always possible.

Moreover, when the waveguide is a scatterer, a scattering coefficient can be used to exponentially vary the propagation of light. Thus, in spite of an exponential variation in the gains of the light receiving sections and the outputs of the light sources, operation checks and correcting operations can be performed.

Furthermore, with an optical fat measuring apparatus according to one embodiment of the present invention, a reflection layer with a specified reflectance is formed in one of the outer surfaces of the waveguide on which the light source and the light receiving section do not face each other. This increases the propagance of the waveguide and enables the volume of the waveguide to be reduced. As a result, the size of the whole apparatus can be reduced.

Moreover, with an optical fat measuring apparatus according to one embodiment of the present invention, a space is provided in a site where the waveguide, the light source section, and the light receiving section face one another. Then, when the light source and the light receiving section are contaminated, the contamination can be prevented from adhering to the light receiving section. Therefore, operation checks and corrections can always correctly be executed.

Furthermore, the light blocking protective cover is connected, via a rotating shaft, to a main body having the light source and light receiving section. Therefore, if the living body is measured, the removed protective cover is unlikely to be lost.

With reference to the drawings, a detailed description will be given below of a method for optically measuring subcutaneous fat and an apparatus used for the method according to the present invention.

### (Embodiment 1)

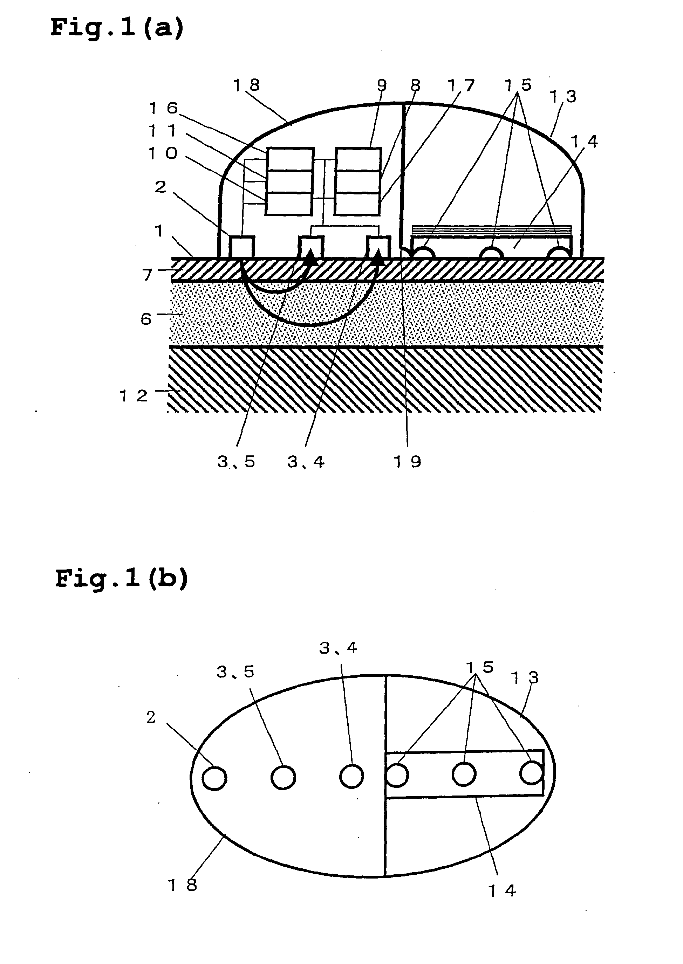

Figure 1(a) is a diagram showing the configuration of an optical fat measuring apparatus according to Embodiment 1 of the present invention. Figure 1(b) is a diagram of the optical fat measuring apparatus according to Embodiment 1 as viewed from a living body.

A light source 2 and a light receiving section 3 are provided on a surface that contacts with a surface 1 of a living body. The light receiving section 3 consists of a measuring light receiving section 4 and a correcting light receiving section 5.

The distance between the measuring light receiving section 4 and the light source 2 is 38 mm. That between the correcting light receiving section 5 and the light source 2 is 23 mm. A port from which light emitted by the light source 2 is emitted has a distance φ of 3 mm. Ports in the measuring light receiving section 4 and correcting light receiving section 5 on which light is incident have a distance φ of 3 mm.

The distance between the measuring light receiving section 4 and the light source 2 is preferably between 35 and 80 mm. The distance between the correcting light receiving section 5 and the light source 2 is preferably between 15 and 30 mm.

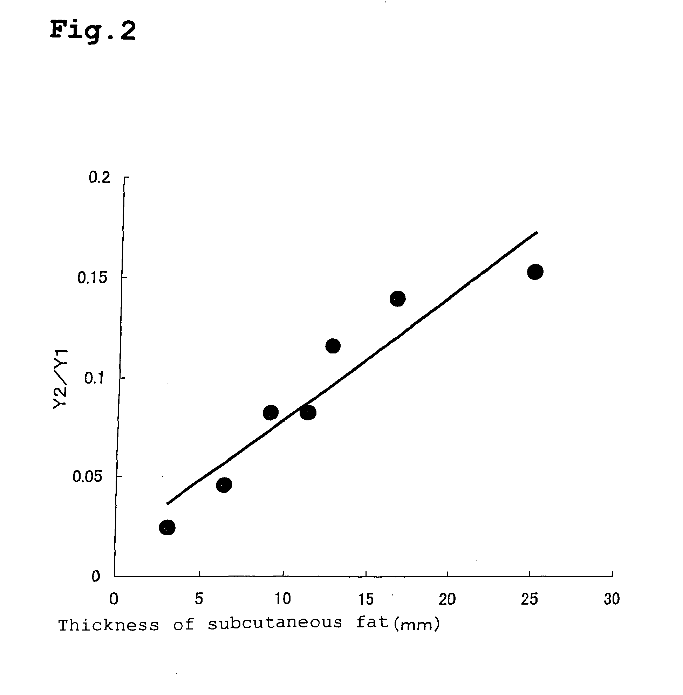

The present apparatus is brought into contact with the living body surface 1. When the light source 2 is lighted, the correcting light receiving section 5 receives a quantity of light to be corrected Y1, while the measuring light receiving section 4 receives a quantity of light to be measured Y2. Figure 2 shows the relationship between the thickness of subcutaneous fat 6 and a parameter Y2/Y1 corresponding to a division of the quantity of received light to be measured Y2 by the quantity of received light to be corrected Y1. In Figure 2, black circles show the relationship between the Y2/Y1 and the thickness of the subcutaneous fat 6. As is apparent from Figure 2, the use of the Y2/Y1 enables the effect on the skin 7 to be corrected and also enables the thickness of the subcutaneous fat 6 to be accurately measured. That is, the use of the Y2/Y1 enables the thickness of the subcutaneous fat 6 to be accurately measured in spite of a variation in the color of the skin 7.

In this case, the light source 2 uses a LED with a central wavelength of 680 nm as a light source element.

The light source element is preferably a laser diode or LED with a central wavelength of 500 to 1,000 nm because of their characteristics showing a large difference in light propagation characteristic between the fat and the muscle.

Moreover, the apparatus is configured so that light is guided from the light source element to the living body surface 1 using a light guiding part such as an optical fiber. This is because heat generated by the light source element is not transmitted to the living body surface.

The light receiving section 3 uses a photo diode as a light receiving element. The light receiving element may be a photoelectric converting element such as CdS. The apparatus is configured so that light is guided from the living body surface 1 to the light receiving element using a light guiding part such as an optical fiber.

An arithmetic section 8 calculates the thickness of the subcutaneous fat 6 on the basis of the Y2/Y1 using the relationship in Figure 2. The calculated thickness of the subcutaneous fat 6 is displayed in the display section 9 and is sent to another equipment as data through a communication section 10.

Further, data such as the height, weight, age, sex, and measured site are inputted directly from an input section 11 or through a communication section 10 from other devices. Then, the arithmetic section 8 calculates the percentage of body fat correlated with the thickness of the subcutaneous fat 6. The percentage of body fat can be displayed on the display section 9 or the data can be transferred to the equipment through the communication section 10.

Further, the optical fat measuring apparatus according to the present embodiment has a light blocking protective cover 13 that covers the light source 2 and the light receiving section 3. A waveguide 14 formed of a scatterer such as high-density polyethylene is placed inside the protective cover 13. Recesses 15 are formed in a site of the waveguide 14 which faces the light source 2 and light receiving section 3 as shown in Figures 1(a) and 1(b). Thus, when the protective cover is closed, the light source 2 and the light receiving section 3 do not contact tightly with the waveguide 14. Such a structure prevents the waveguide 14 from being contaminated even if the light source 2 and the light receiving section 3 are contaminated. Thus, accurate operation checks and corrections are always possible.

Further, the waveguide 14 may be composed of a combination of a first waveguide connecting the light source 2 and the measuring light receiving section 4 and a second waveguide connecting the light source 2 and the correcting light receiving section 5.

Alternatively, the waveguide 14 may be an optical fiber composed of materials such as an infrared (heat ray) absorbing glass which has a specified absorptivity. If the waveguide 14 is an optical fiber, a reduced quantity of light leaking from the waveguide 14 or environmental light such as sunlight enters the waveguide 14. Consequently, the protective cover need not block light.

Furthermore, the scatterer may be polycarbonate. In particular, polycarbonate of a light diffusion grade is useful because diffused rays have an increased transmittance.

Now, description will be given of an operation checking section 16 and a correcting section 17 according to the present invention.

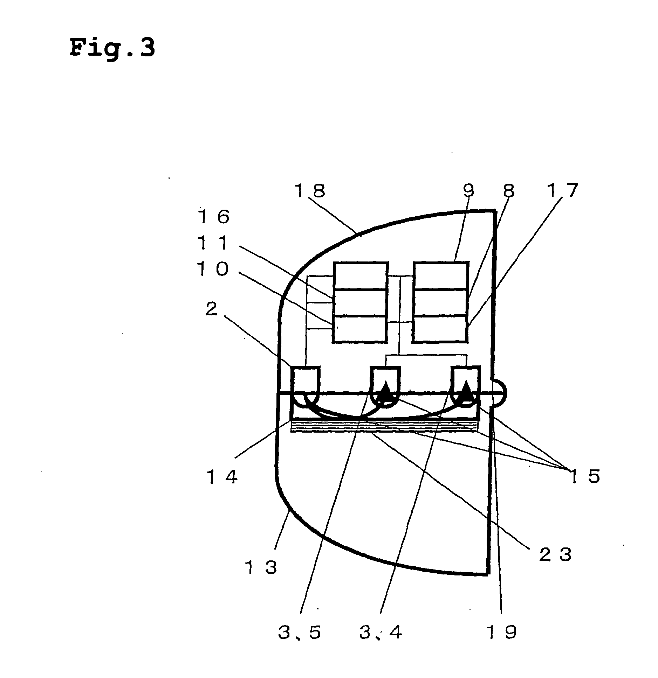

After the protective cover 13 has been mounted, that is, the protective cover 13 has been placed around the light source 2 and the light receiving section 3 with the waveguide 14 placed opposite the light source 2 and light receiving section 3 as shown in Figure 3, the light source 2 is lighted. The light propagates through the waveguide 14 and is then received by the light receiving section 3. The operation checking section 16 compares the quantity of light received by the light receiving section 3 with a reference value predetermined for each light receiving section as a reference. The reference value is prestored in a memory provided in the operation checking section 16.

That is, the operation checking section 16 compares the quantity of light received by the measuring light receiving section 4 with the reference value predetermined for the measuring light receiving section 4 as a reference. The operation checking section 16 also compares the quantity of light received by the correcting light receiving section 5 with the reference value predetermined for the correcting light receiving section 5 as a reference.

Then, if the quantity of light received by one of the light receiving sections is small, the operation checking section 16 determines that this light receiving section is defective. Then, a display section 9 shows that the light receiving section must be cleaned. Once the display section 9 shows that the light receiving section must be cleaned, the user of the optical fat measuring apparatus cleans the light receiving section for which the need for cleaning is displayed. Further, if the quantities of light received by all the light receiving sections are small, the operation checking section 16 determines that the light source 2 is defective. Then, the display section 9 shows that the light source 2 must be cleaned. Once the display section 9 shows that the light source 2 must be cleaned, the user of the optical fat measuring apparatus cleans the light source 2 for which the need for cleaning is displayed. By thus displaying the need for cleaning it is possible to inform the user of the defect and measures to be taken. Accurate fat measurement is always possible.

Before measurement is started and after the protective cover 13 has been installed, that is, the protective cover 13 has been placed around the light source 2 and the light receiving section 3 with the waveguide 14 placed opposite the light source 2 and light receiving section 3 as shown in Figure 3, the light source 2 is lighted. The light propagates through the waveguide 14. The correcting light receiving section 5 receives a quantity of light to be corrected (Y1'), while the measuring light receiving section 4 receives a quantity of light to be measured (Y2'). The correcting section 17 then divides the received light quantity ratio of Y2'/Y1' by the received light quantity ratio of Y2/Y1 obtained when the living body was measured. This corrects degraded accuracy in measured values resulting from a variation in the light intensity of the light source 2 or a variation in the sensitivities of the measuring light receiving section 4 and correcting light receiving section 5 caused by a change in temperature or a secular change. Therefore, accurate fat measurement is always possible.

Further, when the protective cover is connected to the main body 18 by a rotating shaft 19 as shown in Figure 3, the protective cover 13 is prevented from being lost while the fat is being measured. Further, when the cover 20 covers the waveguide 14 in unison with rotation of the rotating shaft 19, the waveguide 14 is not exposed from the apparatus when the living body is measured. This prevents the waveguide 14 from being contaminated during measurement. Therefore, accurate fat measurement is always possible.

Furthermore, when a reflection layer made of aluminum or the like and having a high reflectivity is provided on a surface of the waveguide 14 which does not face the light source 2 and light receiving section 3, the light propagation efficiency of the waveguide 14 increases in spite of the small thickness of the scatterer. It is therefore possible to reduce the thickness and size of the scatterer and thus the size of the apparatus itself.

The light source 2 according to the present embodiment is an example of the light source section according to the present invention. The protective cover 13, waveguide 14, and reflection layer 23 according to the present embodiment are examples of standard elements according to the present invention. The thickness of the subcutaneous fat 6 according to the present embodiment is an example of information on the fat according to the present invention. The percent of body fat correlated with the thickness of the subcutaneous fat 6 according to the present embodiment is an example of information on the fat according to the present invention.

In the description of the present embodiment, the waveguide 14 is a scatterer. However, the waveguide 14 may be an absorber that attenuates light exponentially with a distance. Such an absorber may be an infrared (heat ray) absorbing glass or amaterial for an ND filter. Alternatively, a polycarbonate resin, which absorbs a visible light or a near infrared light, can be used as an absorber.

In the description of the present embodiment, if the operation checking section 16 determines that one of the light receiving sections is defective, the display section 9 shows that the light receiving section determined to be defective must be cleaned. However, the present invention is not limited to this aspect. If the operation checking section 16 determines that one of the light receiving sections is defective, the display section 9 may use an alarm sound such as a buzzer, a voice message, or the like to inform the user of the optical fat measuring apparatus that the light receiving section determined to be defective must be cleaned. Further, in the description, if the operation checking section 16 determines that the light source 2 is defective, the display section 9 shows that the light source 2, determined to be defective, must be cleaned. However, the present invention is not limited to this aspect. If the operation checking section 16 determines that the light source 2 is defective, the display section 9 may turn on an alarm such as a buzzer or use a voice message or the like to inform the user of the optical fat measuring apparatus that the light source 2, determined to be defective, must be cleaned.

Moreover, in the present embodiment, the optical fat measuring apparatus may be used to perform an operation of measuring the living body, and a measuring operation may then be performed after the protective cover 13 has been mounted, that is, after the protective cover 13 has been placed around the light source 2 and the light receiving section 3 with the waveguide 14 placed opposite the light source 2 and light receiving section 3. Alternatively, a measuring operation may performed after the protective cover 13 has been mounted, that is, after the protective cover 13 has been placed around the light source 2 and the light receiving section 3 with the waveguide 14 placed opposite the light source 2 and light receiving section 3 and then the optical fat measuring apparatus may be used to perform an operation of measuring the living body.

Further, if the optical fat measuring apparatus is used to perform an operation of measuring the living body, and a measuring operation is then performed after the protective cover 13 has been closed, that is, after the protective cover 13 has been placed around the light source 2 and the light receiving section 3 with the waveguide 14 placed opposite the light source 2 and light receiving section 3, then after the optical fat measuring apparatus is used to perform the operation of measuring the living body, automatic measurement may be executed when the user closes the protective cover, that is, the protective cover 13 is placed around the light source 2 and light receiving section 3 with the waveguide 14 placed opposite the light source 2 and light receiving section 3. Then, even if the user forgets to perform a measuring operation after closing the protective cover 13, that is, after the protective cover 13 has been placed around the light source 2 and the light receiving section 3 with the waveguide 14 placed opposite the light source 2 and light receiving section 3, automatic measurement is executed while the protective cover 13 remains closed. This enables information on the fat to be accurately determined.

Moreover, in the above description, the optical fat measuring apparatus according to the present embodiment comprises both operation checking section and correcting section. However, the present invention is not limited to this aspect. The optical fat measuring apparatus may be configured to comprise only one of the operation checking section and correcting section.

In the description of the present embodiment, the recesses 15 are formed in the waveguide 14 as shown in Figures 1(a) and 1(b). However, the present invention is not limited to thisaspect. The recesses 15 may not be formed in the waveguide 14 if the configuration is such that when the protective cover 13 is closed, there may be a specified distance between the waveguide 14 and both light source 2 and light receiving section 3 so as to avoid the tight contact between them. In this case, if the distance between the waveguide 14 and both light source 2 and light receiving section 3 is excessively long, light emitted by the light source 2 and impinging on and reflected by the surface of the waveguide 14 may directly enter the light receiving section 3. Accordingly, both light source 2 and light receiving section 3 must be close to the waveguide 14 to the degree that the intensity of light emitted by the light source 2, then impinging on and reflected by the surface of the waveguide 14, and subsequently entering the light receiving section 3 is negligible. Consequently, the specified distance may be such that the both light source 2 and light receiving section 3 is so close to the waveguide 14 that the intensity of light emitted by the light source 2, then impinging on and reflected by the surface of the waveguide 14, and subsequently entering the light receiving section 3 is negligible. In short, after the protective cover 13 has been closed with the waveguide 14 placed opposite the light source 2 and light receiving section 3, it is only necessary that a space is provided between the waveguide 14 and both light source 2 and light receiving section 3, and that the distance between the waveguide 14 and both light source 2 and light receiving section 3 is such as described above.

In the description of the present embodiment, the protective cover 13 is connected to the main body 18 by the rotating shaft 19. However, the present invention is not limited to this aspect. The protective cover 13 may be removable from the main body 18. If the protective cover 13 can be removed from the main body 18, then when for example, the protective cover 13 is cleaned, it can be easily cleaned by being removed from the main body 18. Further, even if the waveguide 14 in the protective cover 13 is broken, it is only necessary to replace the protective cover 13 with a new one. This makes the maintenance of the optical fat measuring apparatus easy.

Moreover, in the description of Embodiment 1, the light receiving section 3 is composed of the measuring light receiving section 4 and the correcting light receiving section 5, that is, the two light receiving sections are provided. However, the present invention is not limited to this aspect. The light receiving section 3 may be composed of three or more light receiving sections. If three or more light receiving sections 3 are provided, the operation checking section 16 operates as described below. The operation checking section 16 compares the quantity of light received by each of the plurality of light receiving sections with a reference value predetermined for each light receiving section as a reference. Then, if the quantity of light received by one of the light receiving sections is small, the operation checking section 16 determines that this light receiving section is defective. Then, the display section 9 shows that the light receiving section must be cleaned. Once the display section 9 shows that the light receiving section must be cleaned, the user of the optical fat measuring apparatus cleans the light receiving section for which the need for cleaning is displayed. Further, if the quantities of light received by all the light receiving sections are small, the operation checking section 16 determines that the light source 2 is defective. Then, the display section 9 shows that the light source 2 must be cleaned. Once the display section 9 shows that the light source 2 must be cleaned, the user of the optical fat measuring apparatus cleans the light source 2 for which the need for cleaning is displayed. By thus displaying the need for cleaning, it is possible to inform the user of the defect and measures to be taken. Accurate fat measurement is always possible as in the case of Embodiment 1.

### (Embodiment 2)

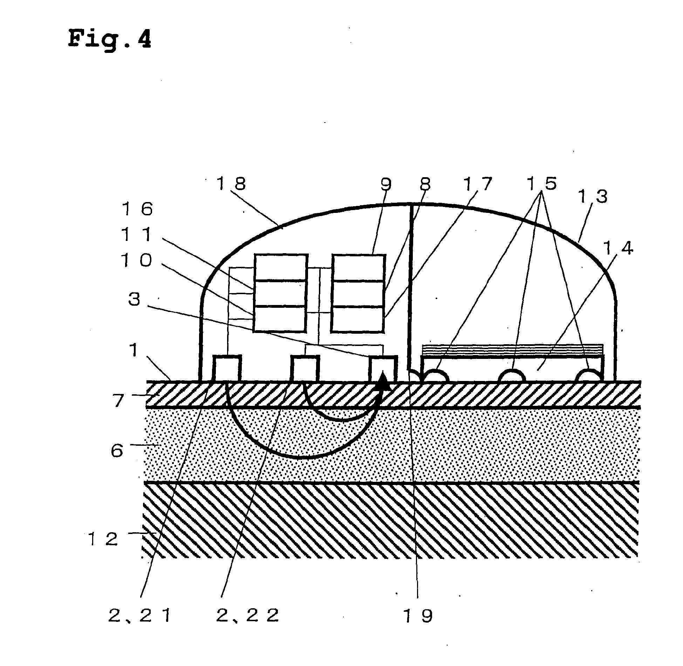

Figure 4 is a diagram showing the configuration of an optical fat measuring apparatus according to Embodiment 2 of the present invention.

Description of parts similar to those of Embodiment 1 is omitted, and different parts will be described.

A plurality of light sources 2 and the light receiving section 3 are provided on a surface that contacts with the surface 1 of the living body. The light sources 2 consist of a measuring light source 21 and a correcting light source 22. The distance between the measuring light source 21 and the light receiving section 3 is 38 mm. That between the correcting light source 22 and the light receiving section 3 is 23 mm. The measuring light source 21, the correcting light source 22, and the light receiving section 3 are lined up almost straight.

The present apparatus is brought into contact with the living body surface 1. When the correcting light source 22 is lighted, the light receiving section 3 receives the quantity of light to be corrected Y1. Then, the measuring light source 21 is lighted, the light receiving section 3 receives the quantity of light to be measured Y2. Figure 2 shows the relationship between the thickness of subcutaneous fat 6 and a parameter Y2/Y1 corresponding to a division of the quantity of received light to be measured Y2 by the quantity of received light to be corrected Y1. In Figure 2, black circles show the relationship between the Y2/Y1 and the thickness of the subcutaneous fat 6. As is apparent from Figure 2, the use of the Y2/Y1 enables the effect on the skin to be corrected and also enables the thickness of the subcutaneous fat to be accurately measured.

Now, description will be given of the operation checking section 16 and correcting section 17 according to the present invention.

After the protective cover 13 has been installed, that is, the protective cover 13 has been placed around the light sources 2 and the light receiving section 3 with the waveguide 14 placed opposite the light sources 2 and light receiving section 3, the operation checking section 16 lights the correcting light source 22. The light propagates through the waveguide 14 and is then received by the light receiving section 3. The operation checking section 16 then measures the quantity of light received by the light receiving section 3, Y1'. Then, the operation checking section 16 lights the measuring light source 21 and measures the quantity of light received by the light receiving section 3, Y2'. The operation checking section 16 then compares the plurality of quantities of light received with reference values determined in association with the respective quantities of light received as references. Then, if only one of the quantities of light received is small, the operation checking section 16 determines that this light source 2 is defective. Then, the display section 9 shows that the light source 2 must be cleaned. Once the display section 9 shows that the light source 2 must be cleaned, the user of the optical fat measuring apparatus cleans the light source 2 for which the need for cleaning is displayed. Further, if the quantity of light received is small for all the light sources 2, the operation checking section 16 determines that the light receiving section 3 is defective. Then, the display section 9 shows that the light receiving section 3 must be cleaned. Once the display section 9 shows that the light receiving section 3 must be cleaned, the user of the optical fat measuring apparatus cleans the light receiving section for which the need for cleaning is displayed. By thus displaying the need for cleaning, it is possible to inform the user of the defect and measures to be taken. Accurate fat measurement is always possible. It is possible to inform the user of the defect and measures to be taken. Accurate fat measurement is always possible.

Before measurement is started and after the protective cover 13 has been installed, that is, the protective cover 13 has been placed around the light source 2 and the light receiving section 3 with the waveguide 14 placed opposite the light source 2 and light receiving section 3, the correcting section 17 lights the correcting light source 22. The light propagates through the waveguide 14. The light receiving section 3 then receives the quantity of light to be corrected Y1'. Then, the correcting section 17 lights the measuring light source 21. The light receiving section 3 then receives the quantity of light to be measured Y2'. The correcting section 17 then divides the received light quantity ratio of Y2'/Y1' by the received light quantity ratio of Y2/Y1 obtained when the living body was measured. This corrects degraded accuracy in measured values resulting from a variation in the sensitivity of the light receiving section 3 or a variation in outputs from the measuring light source 21 and correcting light source 22 caused by a change in temperature or a secular change. Therefore, accurate fat measurement is always possible.

In the description of the present embodiment, if the operation checking section 16 determines that the light receiving section 3 is defective, the display section 9 shows that the light receiving section determined to be defective must be cleaned. However, the present invention is not limited to this aspect. If the operation checking section 16 determines that the light receiving section is defective, the display section 9 may use an alarm sound such as a buzzer, a voice message, or the like to inform the user of the optical fat measuring apparatus that the light receiving section, determined to be defective, must be cleaned. Further, in the description, if the operation checking section 16 determines that the light source 2 is defective, the display section 9 shows that the light source 2 determined to be defective must be cleaned. However, the present invention is not limited to this aspect. If the operation checking section 16 determines that the light source 2 is defective, the display section 9 may turn on an alarm such as a buzzer or use a voice message or the like to inform the user of the optical fat measuring apparatus that the light source 2, determined to be defective, must be cleaned.

Moreover, in the present embodiment, the optical fat measuring apparatus may be used to perform an operation of measuring the living body, and a measuring operation may then be performed after the protective cover 13 has been mounted, that is, after the protective cover 13 has been placed around the light source 2 and the light receiving section 3 with the waveguide 14 placed opposite the light source 2 and light receiving section 3. Alternatively, a measuring operation may performed after the protective cover 13 has been mounted, that is, after the protective cover 13 has been placed around the light source 2 and the light receiving section 3 with the waveguide 14 placed opposite the light source 2 and light receiving section 3, and then the optical fat measuring apparatus may be used to perform an operation of measuring the living body.

Further, if the optical fat measuring apparatus is used to perform an operation of measuring the living body, and a measuring operation is then performed after the protective cover 13 has been closed, that is, after the protective cover 13 has been placed around the light source 2 and the light receiving section 3 with the waveguide 14 placed opposite the light source 2 and light receiving section 3, then after the optical fat measuring apparatus is used to perform the operation of measuring the living body, automatic measurement may be executed when the user closes the protective cover, that is, the protective cover 13 is placed around the light source 2 and light receiving section 3 with the waveguide 14 placed opposite the light source 2 and light receiving section 3. Then, even if the user forgets to perform a measuring operation after closing the protective cover 13, that is, after the protective cover 13 has been placed around the light source 2 and the light receiving section 3 with the waveguide 14 placed opposite the light source 2 and light receiving section 3, automatic measurement is executed while the protective cover 13 remains closed. This enables information on the fat to be accurately determined.

In the description of the present embodiment, the recesses 15 are formed in the waveguide 14 as shown in Figures 1(a) and 1(b). However, the present invention is not limited to this aspect. The recesses 15 may not be formed in the waveguide 14 if the configuration is such that when the protective cover 13 is closed, there may be a specified distance between the waveguide 14 and both light source 2 and light receiving section 3 so as to avoid the tight contact between them. In this case, if the distance between the waveguide 14 and both light source 2 and light receiving section 3 is excessively long, light emitted by the light source 2 and impinging on and reflected by the surface of the waveguide 14 may directly enter the light receiving section 3. Accordingly, both light source 2 and light receiving section 3 must be close to the waveguide 14 to the degree that the intensity of light emitted by the light source 2, then impinging on and reflected by the surface of the waveguide 14, and subsequently entering the light receiving section 3 is negligible. Consequently, the specified distance may be such that the both light source 2 and light receiving section 3 is so close to the waveguide 14 that the intensity of light emitted by the light source 2, then impinging on and reflected by the surface of the waveguide 14, and subsequently entering the light receiving section 3 is negligible. In short, after the protective cover 13 has been closed with the waveguide 14 placed opposite the light source 2 and light receiving section 3, it is only necessary that a space is created between the waveguide 14 and both light source 2 and light receiving section 3, and that the distance between the waveguide 14 and both light source 2 and light receiving section 3 is such as described above.

In the description of the present embodiment, the protective cover 13 is connected to the main body 18 by the rotating shaft 19. However, the present invention is not limited to this aspect. The protective cover 13 may be removable from the main body 18. If the protective cover 13 can be removed from the main body 18, then when for example, the protective cover 13 is cleaned, it can be easily cleaned by being removed from the main body 18. Further, even if the waveguide 14 in the protective cover 13 is broken, it is only necessary to replace the protective cover 13 with a new one. This makes the maintenance of the optical fat measuring apparatus easy.

Moreover, in the description of Embodiment 2, the light source 2 is composed of the measuring light source 21 and the correcting light source 22, that is, the two light sources are provided. However, the present invention is not limited to this aspect. The light source 2 may be composed of three or more light sources. If three or more light sources 2 are provided, the operation checking section 16 operates as described below. The operation checking section 16 compares the quantity of received light emitted by each of the plurality of light sources with a reference value predetermined for each light source as a reference. Then, if the quantity of light received for only one of the light sources is small, the operation checking section 16 determines that this light source is defective. Then, the display section 9 shows that the light source must be cleaned. Once the display section 9 shows that the light source must be cleaned, the user of the optical fat measuring apparatus cleans the light source for which the need for cleaning is displayed. Further, if all the quantities of received light emitted by all the light sources are small, the operation checking section 16 determines that the light receiving section 3 is defective. Then, the display section 9 shows that the light receiving section 3 must be cleaned. Once the display section 9 shows that the light receiving section must be cleaned, the user of the optical fat measuring apparatus cleans the light receiving section for which the need for cleaning is displayed. By thus displaying the need for cleaning, it is possible to inform the user of the defect and measures to be taken. Accurate fat measurement is always possible as in the case of Embodiment 1.

Thus, according to the present embodiment, it is possible to provide an apparatus and method for optically measuring fat which apparatus and method can correct secular changes and changes in temperature to accurately measure fat.

### Industrial Applicability

As is apparent from the above description, the present invention can provide an optical fat measuring apparatus and a standard element which can correct an error in measurement caused by a change in temperature or a secular change by checking the sensitivities of a light source and a light receiving element.

Further, the present invention can provide an optical fat measuring apparatus and a standard element which can always accurately and reproducibly measure fat by sensing that the light source or the light receiving element is contaminated to display the need for cleaning.

## Claims

1. An optical fat measuring apparatus comprising:
a light source section and a light receiving section arranged so as to obtain a plurality of transmitted to received light distances;
an arithmetic section which calculates information on fat in a living body on the basis of the quantity of light received by said light receiving section, the light being emitted by said light source section and propagating through the living body; and
a standard element having a waveguide which guides light from said light source section to said light receiving section when placed opposite said light source section and said light receiving section, said waveguide having a predetermined transmittance, and
wherein at least one of said light source section and said light receiving section, is arranged plurally.

2. The optical fat measuring apparatus according to Claim 1, having one of said light source sections and a plurality of said light receiving sections, and
wherein said apparatus comprises an operation checking section which compares a reference value for the quantity of light received predetermined for each said light receiving section with the quantity of received light which has been guided from lighted said light source section to the plurality of said light receiving sections through said waveguide after said standard element has been placed so that said waveguide is opposite said light source section and said light receiving sections,
which operates if the quantities of light received by a smaller number of said light receiving sections than that of the plurality of said light receiving sections are smaller than said reference value for the quantity of light received corresponding to these light receiving sections, to determine that these light receiving sections are defective, and
which operates if each quantities of light received by all of the plurality of said light receiving sections are smaller than the reference value for the quantity of light received corresponding to said each light receiving sections, to determine that said light source section is defective.

3. The optical fat measuring apparatus according to Claim 1, wherein if said operation checking section determines that said light receiving section is defective, said operation checking section shows that said light receiving section determined to be defective must be cleaned or provides a corresponding sound output, and
if said operation checking section determines that said light source section is defective, said operation checking section shows that said light source section determined to be defective must be cleaned or provides a corresponding sound output.

4. The optical fat measuring apparatus according to Claim 1, having a plurality of said light source sections and one said light receiving section, and
wherein the apparatus comprises an operation checking section which compares a reference value for the quantity of light received predetermined for each said light source section with the quantity of received light which has been guided from the independently lighted plurality of said light source sections to said light receiving section through said waveguide after said standard element has been placed so that said waveguide is opposite said light source sections and said light receiving section,
which operates if the quantities of light received from a smaller number of said light source sections than that of the plurality of said light source sections are smaller than said reference value for the quantity of light received corresponding to these light source sections, to determine that these light source sections are defective, and
which operates if each quantities of light received from all of the plurality of said light source sections are smaller than the reference value for the quantity of light received corresponding to said each light source sections, to determine that said light receiving section is defective.

5. The optical fat measuring apparatus according to Claim 1, wherein if said operation checking section determines that said light source section is defective, said operation checking section shows that said light source section determined to be defective must be cleaned or provides a corresponding sound output, and
if said operation checking section determines that said light receiving section is defective, said operation checking section shows that said light receiving section determined to be defective must be cleaned or provides a corresponding sound output.

6. The optical fat measuring apparatus according to Claim 1, having one of said light source section and a plurality of said light receiving sections, and
wherein said arithmetic section has a correcting section which corrects measured values for said living body using the quantity of received light which has been guided from lighted said light source section to the plurality of said light receiving sections through said waveguide after said standard element has been placed so that said waveguide is opposite said light source section and said light receiving sections.

7. The optical fat measuring apparatus according to Claim 1, having a plurality of said light source sections and one said light receiving section, and
wherein said arithmetic section has a correcting section which corrects measured values for said living body using the quantity of received light which has been guided from independently lighted plurality of said light source sections to said light receiving section through said waveguide after said standard element has been placed so that said waveguide is opposite said light source sections and said light receiving section.

8. The optical fat measuring apparatus according to Claim 6, wherein said light receiving section includes a first light receiving section and a second light receiving section, and
after said standard element has been placed so that said waveguide is opposite said light source section and said light receiving sections, said correcting section corrects the measured values for said living body on the basis of the ratio of the quantity of light received by said first light receiving section, the light being guided from lighted said light source and to said first light receiving section through said waveguide, to the quantity of light received by said second light receiving section, the light being guided from lighted said light source to said second light receiving section through said waveguide.

9. The optical fat measuring apparatus according to Claim 7, wherein said light source section includes a first light source section and a second light source section, and
after said standard element has been placed so that said waveguide is opposite said light source sections and said light receiving section, said correcting section corrects the measured values for said living body on the basis of the ratio of the quantity of received light guided from independently lighted said first light source to said light receiving section through said waveguide, to the quantity of received light guided from independently lighted said second light source to said light receiving section through said waveguide.

10. The optical fat measuring apparatus according to any one of Claims 1 to 9, wherein said standard element is connected by a rotating shaft to a main body with said light source section and said light receiving section.
